# EUROPÄISCHE PATENTSCHRIFT

(11) **EP 0 864 559 B1**
(45) Veröffentlichungstag und Bekanntmachung des Hinweises auf die Patenterteilung: **05.06.2002**
(21) Anmeldenummer: 98102853.3
(22) Anmeldetag: 19.02.1998
(51) Int. Cl.: C07C 215/54, C07C 215/64, C07C 211/28, A61K 31/135

(54) **Substituierte Aminoverbindungen und ihre Verwendung als analgetisch wirksame Substanzen**
Substituted amino compounds and their use as analgesic active substances
Composés amino substitués et leur utilisation comme substances actives analgésiques

(30) Priorität: 14.03.1997 DE 19710628
(43) Veröffentlichungstag der Anmeldung: 16.09.1998
(73) Patentinhaber: Grünenthal GmbH, 52078 Aachen (DE)
(72) Erfinder: Zimmer, Oswald, Dr., 52146 Würselen (DE); Strassburger, Wolfgang Werner Alfred, Prof. Dr., 52146 Würselen (DE); Buschmann, Helmut Heinrich, Dr., 52066 Aachen (DE); Englberger, Werner, Dr., 52223 Stolberg (DE); Friderichs, Elmar Josef, Dr., 52223 Stolberg (DE)

(56) Entgegenhaltungen:
- BE-A- 623 394
- DE-B- 1 149 002
- DE-B- 1 203 247
- ROBERT BRUCE MOFFETT ET AL.: "a MILD METHOD FOR DEHYDRATING SOME CARBINOLS TO ALKENES. 1,1-dIARYLAMINOALKENES AS ANALGESICS" ORGANIC PREPARATIONS AND PROCEDURES INTERNATIONAL, Bd. 11, Nr. 1, Februar 1979, Seiten 53-61, XP002062236
- D. I. BARRON ET AL.: "Compounds affecting the central nervous system." JOURNAL OF MEDICINAL CHEMISTRY., Bd. 8, Nr. 6, November 1965, WASHINGTON US, Seiten 836-841, XP002062237
- CORNELIS GERRIT WARINGA ET AL.: "1,1-diaryl-3-aminopropenes and some related compounds" EUROPEAN JOURNAL OF MEDICINAL CHEMISTRY.CHIMICA THERAPEUTICA., Bd. 10, Nr. 4, 1975, PARIS FR, Seiten 343-348, XP002062238
- CHEMICAL ABSTRACTS, vol. 72, no. 11, 16.März 1970 Columbus, Ohio, US; abstract no. 54913e, KAMETANI, TETSUJI ET AL.: "Hypertensive agents" Seite 368; Spalte 2; XP002062240 & YAKUGAKU ZASSHI , Bd. 89, Nr. 11, 1969, Seiten 1613-1616,
- J. F. CAVALLA ET AL.: "compounds derived from the Mannich bases of Beta-phenylpropiophenone" JOURNAL OF MEDICINAL CHEMISTRY., Bd. 7, Nr. 6, November 1964, WASHINGTON US, Seiten 716-721, XP002062239

## Beschreibung

Die Erfindung betrifft substituierte Aminoverbindungen der allgemeinen Formel (I) 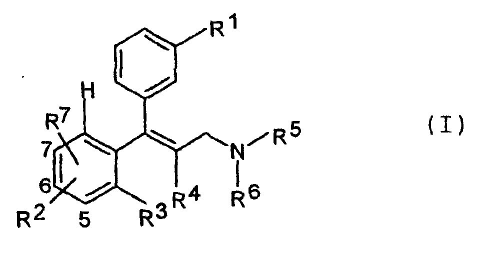 oder pharmazeutisch verträgliche Salze davon, ein Verfahren zu deren Herstellung sowie ihre Verwendung als Arzneimittel.

Klassische Opioide wie das Morphin sind bei der Therapie starker bis stärkster Schmerzen gut wirksam. Ihr Einsatz wird jedoch durch die bekannten Nebenwirkungen, z. B. Atemdepression, Erbrechen, Sedierung und Obstipation und die Toleranzentwicklung eingeschränkt. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Protein-gekoppelten Rezeptoren gehören. Die biochemische und pharmakologische Charakterisierung von Subtypen dieser Rezeptoren hat nun die Hoffnung geweckt, daß subtypenspezifische Opioide über ein anderes Wirkungs-/Nebenwirkungsprofil als z.B. Morphin verfügen. Während Morphin selektiv an die sogenannten µ-Rezeptoren bindet, wurden die endogenen Enkephaline als δ selektive Peptide charakterisiert. Weitere pharmakologische Untersuchungen haben inzwischen die Existenz mehrerer Subtypen dieser Opioidrezeptoren (µ₁ µ₂, κ₁, κ₂, κ₃, δ₁ und δ₂) wahrscheinlich gemacht.

Kenntnisse über die physiologische Bedeutung von δ-rezeptorselektiven Substanzen sind wesentlich durch die Entdeckung des nicht-peptidischen Antagonisten Naltrindol erweitert worden. So steht inzwischen fest, daß δ-Agonisten über ein eigenständiges antinociceptives Potential verfügen. Neben einer Vielzahl von tierexperimentellen Studien gibt es dazu auch eine Untersuchung mit dem peptidischen Agonisten DADL an Krebspatienten, bei denen Morphin keine analgetische Wirkung mehr hatte. Bei intrathekaler Gabe zeigte DADL einen lang anhaltenden analgetischen Effekt.

Deutlich unterscheiden sich δ- von µ-Agonisten in ihrer Wechselwirkung mit dem "endogenen Opioidantagonisten" Cholecystokinin (CCK).

Neben diesem unterschiedlichen Wirkprofil könnte sich auch das Nebenwirkungsprofil der δ-Agonisten von µ-Agonisten unterscheiden, z.B. durch eine geringere Atemdepression.

Im Stand der Technik (Organic Preparations and Prodedures Int. 11(2), 53-61 (1979)) sind symmetrisch substituierte 1,1-Diarylaminoalkene als Analgetika beschrieben.

Die der Erfindung zugrundeliegende Aufgabe bestand deshalb darin, analgetisch wirksame Substanzen zu finden, deren biologische Wirksamkeit teilweise oder überwiegend über δ-Opiatrezeptoren vermittelt wird.

Es wurde nun gefunden, daß diese Anforderungen von den Aminoverbindungen der allgemeinen Formel (I) erfüllt werden.

Gegenstand der Erfindung sind dementsprechend substituierte Aminoverbindungen der allgemeinen Formel (I) 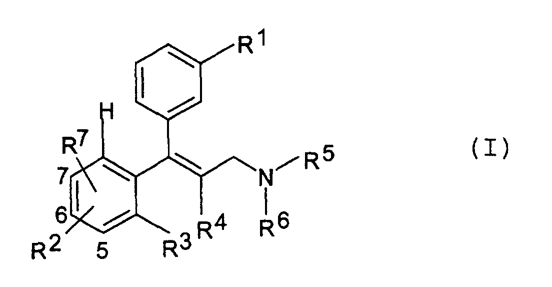 worin
- R¹: H, OH, O-C₁₋₆-Alkyl, O-C₃₋₇-Cycloalkyl; (wobei: R¹ ungleich H, wenn R² und R⁷ gleich H oder in 7-Stellung eine OCH₃-Gruppe substituiert ist);
- R²: H, OH, C₁₋₆-Alkyl, O-Aryl, C₂₋₆-Alkenylen-Aryl, O-C₃₋₇-Cycloalkyl, Cl, F, C₁₋₆-Alkoxy, Aryl (wobei Aryl unsubstituierte oder ein- oder mehrfach mit OH, F, Cl, CF₃, C₁₋₆-Alkyl, C₁₋₆-Alkoxy, Cycloalkoxy mit bis zu 7-C-Atomen, C₃₋₇-Cycloalkyl, C₂₋₆-Alkylen oder Phenyl substituierte Phenyle bedeutet, wobei wiederum die Phenylreste ankondensiert sein können, oder Aryl auch Naphtyl bedeutet), C₁₋₆-Alkyl-Aryl, 5,6- oder 6,7-Benzo-(an)kondensiert unsubstituiert, mono- oder disubstituiert mit Cl, F, CF₃, C₁₋₆-Alkyl, O-C₁₋₆-Alkyl, OH, (wobei: wenn R¹ und R² oder R⁷ gleich H ist,kann R² oder R⁷ keine CF₃-Gruppe und R² oder R⁷ in 7-Stellung keine OCH₃-Gruppe darstellen),
oder R² und R⁷ zusammen -O-(CH₂)₍₁₋₂₎-O- (in 5,6- oder 6,7-Position);
- R³: H;
- R⁴: C₁₋₆-Alkyl, oder R³ und R⁴ zusammen -(CH₂)₍₁₋₄₎-;
- R⁵: C₁₋₆-Alkyl, C₃₋₇-cycloalkyl;
- R⁶: C₁₋₆-Alkyl, C₁₋₆-Alkyl-Aryl; C₁₋₆-Alkyl-Heterocyclyl, wobei Heterocyclyl Furan, Thiophen, Pyridin, Pyrimidin, Thiazol, Oxazol, Isoxazol, Pyridazin, Pyrazin, Chinolin, Isochinolin, Phthalazin oder Chinazolin bedeutet, -CH₂-CH=C(R⁸)₂, -CH₂-(C₃₋₇)-Cycloalkyl, C₃₋₇-Cycloalkyl;
- R⁷: H, OH, C₁₋₆-Alkyl, O-Aryl, C₂₋₆-Alkenylen-Aryl, O-C₃₋₇-Cycloalkyl, C₁₋₆-Alkoxy, Cl, F, Aryl, C₁₋₆-Alkyl-Aryl, 5,6- oder 6,7-Benzo-(an)kondensiert unsubstituiert, mono-oder disubstituiert mit Cl, F, CF₃, C₁₋₆-Alkyl, O-C₁₋₆-Alkyl, OH,
(wobei: wenn R¹ und R² oder R⁷ gleich H ist,kann R² oder R⁷ keine CF₃-Gruppe und R² oder R⁷ in 7-Stellung keine OCH₃-Gruppe darstellen);
und
- R⁸: H oder CH₃
bedeuten, oder pharmazeutische verwendbare Salze davon.

Zu den bevorzugten substituierten Aminoverbindungen der allgemeinen Formel I zählen solche, worin
R⁶ C₁₋₆-Alkyl-Aryl oder C₁₋₆-Alkyl-Heterocyclyl, wobei Heterocyclyl die oben angegebene Bedeutung hat, und R² und R⁷ C₁₋₆-Alkyl-Aryl und R¹, R³ bis R⁵ und R⁸ die oben angegebene Bedeutung der allgemeinen Formel I oder
R², R⁷ Aryl und R¹, R³ bis R⁶ und R⁸ die oben angegebene Bedeutung der allgemeinen Formel I oder
R¹ OH oder -O-C₁₋₆-Alkyl, R⁵ und R⁶ C₁₋₆-Alkyl bedeuten, R² bis R⁴ und R⁷ die weiter oben erwähnte Bedeutung haben.

Besonders bevorzugt sind Verbindungen worin R¹ OH, R⁵ und R⁶ Methyl, R² bis R⁴ und R⁷ die Bedeutung gemäß der allgemeinen Formel I besitzen.

Der Ausdruck "C₁₋₆-Alkyl" bedeutet in der vorliegenden Erfindung geradkettige oder verzweigte Kohlenwasserstoffe mit 1-6 Kohlenstoffatomen. Beispielhaft seien Methyl, Ethyl, Propyl, Isopropyl, n-Butyl, sek-Butyl, tert-Butyl, n-Pentyl, Neopentyl und n-Hexyl genannt.

Der Ausdruck "C₁₋₆-Alkoxy" bedeutet im Rahmen der vorliegenden Erfindung geradkettige oder verzweigte Kohlenwasserstoffe mit 1-6 Kohlenstoffatomen wie oben definiert, die über das Sauerstoffatom gebunden sind.

Der Ausdruck "Aryl" bedeutet im Rahmen der vorliegenden Erfindung unsubstituierte oder ein- oder mehrfach mit OH, F, Cl, CF₃, C₁₋₆-Alkyl, C₁₋₆-Alkoxy, C₁₋₇-Cycloalkoxy, C₃₋₇-Cycloalkyl, C₂₋₆-Alkylen, Heterocyclyl oder Phenyl substituierte Phenyle. Die Heterocyclyloder Phenylreste können gegebenenfalls ankondensiert sein. Der Ausdruck kann gegebenenfalls auch Naphthyl bedeuten.

Unter dem Ausdruck "Heterocyclyl" sind im Rahmen der vorliegenden Erfindung 5- oder 6-gliedrige gesättigte oder ungesättigte, gegebenenfalls mit einem ankondensierten Arylsystem versehene, heterocyclische Verbindungen zu verstehen, die 1 oder 2 Heteroatome aus der Gruppe von Stickstoff, Sauerstoff und/oder Schwefel enthalten.

Beispielhaft seien für die gesättigten Heterocyclyle 1,4-Dioxan, Tetrahydrofuran und 1,4-Thioxan aufgeführt.

Aus der Gruppe der ungesättigten Heterocyclyle seien beispielhaft Furan, Thiophen, Pyridin, Pyrimidin, Thiazol, Oxazol, Isoxazol, Pyridazin, Pyrazin, Chinolin, Isochinolin, Phthalazin und Chinazolin aufgeführt.

Die Ausdrücke "C₁₋₆-Alkyl-Aryl" bzw. "C₁₋₆-Alkyl-Heterocyclyl" bedeuten im Rahmen der vorliegenden Erfindung, daß die zAryle" bzw. "Heterocyclyle" wie oben definiert über eine C₁₋₆-Alkyl-Gruppe gebunden sind.

Unter dem Ausdruck "Silanylverbindung" sind im Rahmen der vorliegenden Erfindung Trialkyl- oder Triarylsilyle, Dialkylarylsilyle oder Diarylalkylsilyle zu verstehen, die als Schutzgruppe für die Hydroxy-Funktion verwendet werden. Beispielhaft seien Triethylsilyl, Tripropylsilyl, Dimethyl-phenylsilyl, Di-tert-butylphenylsilyl, Triiso-propylsilyl, Dimethylisopropylsilyl, Diethylisopropylsilyl, Dimethylthexylsilyl, tert-Butyldimethylsilyl, tert-Butyldiphenylsilyl, Tribenzylsilyl, Tri-p-xylylsilyl, Triphenylsilyl, Diphenylmethylsilyl oder Propyl-diphenylsilyl erwähnt.

Erfindungsgegenstand ist auch ein Verfahren zur Herstellung der substituierten Aminoverbindungen der allgemeinen Formel (I), das gekennzeichnet ist durch die Umsetzung eines tertiären Alkohols der allgemeinen Formel (II) 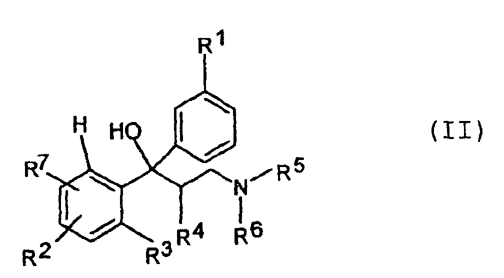 wobei R¹ bis R⁷ dieselbe Bedeutung wie in Formel (I) haben, mit halbkonzentrierten oder konzentrierten organischen oder anorganischen Säuren, insbesondere Ameisensäure oder Salzsäure in einem Temperaturbereich von 0° C und 100° C, wobei die tertiären Alkohole der allgemeinen Formel II, dadurch erhalten werden, daß β-Aminoketone der allgemeinen Formel (III) 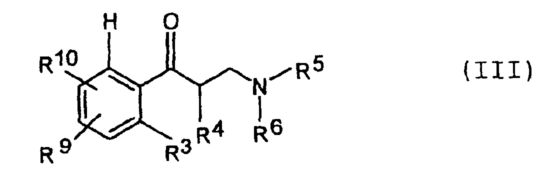 wobei R³ bis R⁶ dieselbe Bedeutung wie in Formel (I) besitzen und R⁹ wie R² und R¹⁰ wie R⁷ definiert ist, mit der Ausnahme, daß die Hydroxy-Funktion in geschützter Form z. B. als Benzyloxy- oder Silanyloxygruppe vorliegt und mit einer metallorganischen Verbindung der Formel (IV) 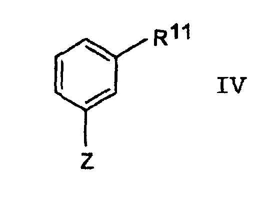 in der Z für MgCl, MgBr, MgI oder Li steht und R¹¹ wie R¹ definiert ist, mit der Ausnahme, daß die Hydroxy-Funktion in geschützter Form als Benzyloxy- oder Silanyloxygruppe wie z. B. tert-Butyldiphenylsilyloxy, die zu einer Verbindung der Formel IIa umgesetzt werden 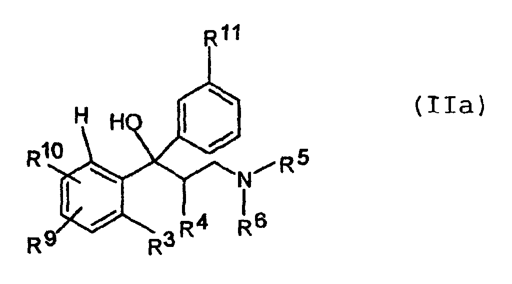 und diese dann in die Verbindung der Formel (II) überführt werden.

Die Umsetzung der Verbindungen (III) und (IV) wird in einem aliphatischen Ether, beispielsweise Diethylether und/oder Tetrahydrofuran, bei Temperaturen zwischen -70°C und +60°C durchgeführt. Verbindungen der Formel (IV), in denen Z für ein Lithiumatom steht, werden dabei aus Verbindungen der Formel (IV), in denen Z Br oder I bedeutet, durch Halogen-Lithiumaustausch mittels z.B. einer n-Butyllithium/n-Hexan-Lösung gewonnen.

Für die Umsetzung einer Verbindung der Formel (IIa) zu einer der Formel (II) stehen in Abhängigkeit von R⁹, R¹⁰ bzw. R¹¹ mehrere Methoden zur Verfügung.

Stehen R⁹, R¹⁰ und/oder R¹¹ für eine Benzyloxygruppe, so geschieht dies zweckmäßig durch eine reduktive Debenzylierung mit katalyisch aktiviertem Wasserstoff, wobei Platin oder Palladium, absorbiert auf einem Trägermaterial wie Aktivkohle, als Katalysator dienen. Die Reaktion wird in einem Lösungsmittel wie Essigsäure oder einem C₁₋₄-Alkylalkohol bei Drücken von 1 bis 100 bar und Temperaturen von +20° C bis +100° C durchgeführt, wobei die Verbindung (IIa) vorzugsweise in Form eines ihrer Salze eingesetzt wird.

Stellen R⁹, R¹⁰ und/oder R¹¹ eine Silylgruppe dar, so erfolgt die Abspaltung der Schutzgruppe dadurch, daß man die entsprechende Verbindung der Formel (IIa) bei +20°C in einem inerten Lösungsmittel wie z. B. Tetrahydrofuran, Dioxan oder Diethylether mit Tetra-n-butylammoniumfluorid umsetzt oder mit einer methanolischen Lösung von Chlorwasserstoff behandelt.

Stehen R⁹, R¹⁰ und/oder R¹¹ in der Verbindung der Formel (IIa) für den Methoxyrest, läßt sich durch Umsetzung mit Diisobutylaluminiumhydrid in einem aromatischen Kohlenwasserstoff wie z. B. Toluol oder Xylol bei einer Temperatur zwischen 60° C und 130° C die Verbindung der Formel (II) herstellen, in der R¹ für eine Hydroxygruppe steht. Man kann auch direkt die analoge Verbindung der Formel (I) erhalten, indem man IIa entweder mit einer Lösung von Bromwasserstoff in Eisessig oder konzentrierter Bromwasserstoffsäure zum Rückfluß erhitzt.

Auch aus Verbindungen der Formel (I), in denen R¹ und/oder R² und/oder R⁷ für eine Methoxygruppe stehen, lassen sich wie oben beschrieben durch Umsetzung mit Diisobutylaluminiumhydrid Verbindungen der Formel I mit R¹, R² und/oder R⁷ gleich OH erhalten.

Die Verbindungen der Formel (I) lassen sich mit physiologisch verträglichen Säuren wie Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure in an sich bekannter Weise in ihre Salze überführen. Vorzugsweise wird die Salzbildung in einem Lösungsmittel wie Diethylether, Diisopropylether, Essigsäurealkylester, Aceton und/oder 2-Butanon durchgeführt. Zur Herstellung der Hydrochloride ist Trimethylchlorsilan in wasserhaltiger Lösung besonders geeignet.

Darüber hinaus ist die Verwendung der erfindungsgemäßen substituierten Aminoverbindungen der allgemeinen Formel (I) als Wirkstoff in Arzneimitteln Gegenstand der Erfindung. Die analgetisch wirksamen Formulierungen enthalten neben mindestens einer erfindungsgemäßen Verbindung der Formel (I) Hilfsstoffe, beispielsweise Trägermittel, Lösungsmittel, Verdünnungsmittel, Farbstoffe und Bindemittel. Die Auswahl dieser Hilfsstoffe sowie die einzusetzenden Mengen derselben hängt davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parentale, topische und inhalative Applikation Lösungen, Suspensionen, leicht rekonstituierbare Trockenzubereitungen sowie Sprays. Erfindungsgemäße Verbindungen in einem Depot in gelöster Form, einer Trägerfolie oder einem Pflaster, gegebenenfalls unter Zusatz von die Hauptpenetration fördernden Mitteln, sind Beispiele für geeignete perkutane Applikationsformen. Aus oral oder perkutan anwendbaren Zubereitungsformen können die erfindungsgemäßen Verbindungen verzögert freigesetzt werden. Die an Patienten zu verabreichende Wirkstoffmenge variiert in Abhängigkeit vom Gewicht des Patienten, von der Applikationsart, der Indikation und dem Schweregrad der Erkrankung.

Die nachfolgenden Beispiele dienen zur näheren Erläuterung des erfindungsgemäßen Verfahrens.

Als stationäre Phase für die Säulenchromatographie wurde Kieselgel 60 (0.040 - 0.063 mm) der Firma E. Merck, Darmstadt, eingesetzt.

Die dünnschichtchromatographischen Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

### Beispiel 1

### 3-(2-Dimethylaminomethyl-3,4-dihydro-naphth-1-yl)-phenol, Hydrochlorid

### 1. Stufe:

### (RS)-2-Dimethylaminomethyl-3,4-dihydro-2H-naphthalin-1-on

Eine Lösung von 21 ml 3,4-Dihydro-2H-naphthalin-1-on in 200 ml Eisessig wurde nacheinander mit 8,2 g Dimethylamin, Hydrochlorid und 3,0 g Paraformaldehyd versetzt. Die Mischung wurde 2 Stunden auf 100°C erhitzt, danach wurde im Vakuum das Lösungsmittel abgedampft und der Rückstand in 200 ml Wasser aufgenommen. Man extrahierte dreimal mit je 100 ml Diethylether. Die wäßrige Phase wurde unter kräftigem Rühren durch portionsweise Zugabe von Kaliumcarbonat auf pH 10 gebracht. Dann wurde dreimal mit je 150 ml Essigsäureethylester extrahiert. Die Extrakte wurden mit einer gesättigten Natriumchloridlösung gewaschen und über Natriumsulfat getrocknet. Nach Abfiltrieren und Eindampfen des Filtrats im Vakuum blieben 15,4 g (75,6 % d.Th.) (RS)-2-Dimethylaminomethyl-3,4-dihydro-2H-naphthalin-1-on als gelbliches Öl zurück.

### 2. Stufe:

### (1RS, 2RS)-2-Dimethylaminomethyl-1-(3-methoxy-phenyl)-1,2,3,4-tetrahydro-naphth-1-ol

Eine Lösung von 7,5 g 1-Brom-3-methoxy-benzol in 15 ml trockenem Tetrahydrofuran wurde bei -50°C unter Rühren und N₂-Schutzgasatmosphäre tropfenweise mit 25 ml einer 1,6-molaren Lösung von n-Butyllithium in n-Hexan versetzt. Man rührte 30 Minuten bei -30°C nach und tropfte dann bei -50°C eine Lösung von 6,1 g des Produkts aus Stufe 1 in 120 ml trockenem Tetrahydrofuran zu. Die Mischung wurde danach 3 Stunden bei -50°C, dann 12 Stunden bei -20°C gerührt. Nach Zugabe von 100 ml Salzsäure (10 %) wurde zweimal mit je 100 ml Essigsäureethylester extrahiert. Die salzsaure Phase wurde durch Zugabe von Kaliumcarbonat auf ca. pH 10 gebracht und dreimal mit je 50 ml Dichlormethan extrahiert. Die Extrakte trocknete man über Natriumsulfat, dampfte das Lösungsmittel im Vakuum ab und reinigte den Rückstand säulenchromatographisch mit Essigsäureethylester /Methanol = 3/1 als Elutionsmittel. Dabei fielen 5,3 g (56,5 % d. Th.) (1RS, 2RS)-2-Dimethylaminomethyl-1-(3-methoxy-phenyl)-1,2,3,4-tetrahydro-naphth-1-ol in Form eines viskosen Öls an.

### 3. Stufe:

### 3- (2-Dimethylaminomethyl-3,4-dihydro-naphth-1-yl)-phenol, Hydrochlorid

5,2 g des Produkts aus Stufe 2 wurden mit 160 ml einer Lösung von Bromwasserstoff in Eisessig (33 % HBr) 6 Stunden zum Rückfluß erhitzt. Dann wurde im Vakuum eingedampft und der Rückstand in 150 ml Wasser aufgenommen. Es wurde mit Natriumcarbonat alkalisiert und dreimal mit je 50 ml Dichlormethan extrahiert. Nach Waschen der Extrakte mit einer gesättigten Natriumchloridlösung und Trocknen über Natriumsulfat wurde eingedampft und der Rückstand säulenchromatographisch mit Essigsäureethylester/Methanol = 5/1 als Elutionsmittel gereinigt. Die so erhaltene Base der Titelverbindung wurde mit Trimethylchlorsilan/Wasser in 2-Butanon ins Hydrochlorid übergeführt.
Ausbeute: 2,3 g (43,8 % d.Th.)
Schmelzpunkt: 197 - 199°C

### Beispiel 2

Unter Einsatz von Indan-1-on, 3,4-Dihydro-2H-phenanthren-1-on, 6,7,8,9-Tetrahydro-benzocyclohepten-5-on, 7,8,9,10-Tetrahydro-6H-benzocycloocten-5-on, 5-Phenyl-3,4-dihydro-2H-naphthalin-1-on, 6-Phenyl-3,4-dihydro-2H-naphthalin-1-on, 6-(3-Chlor-phenyl)-3,4-dihydro-2H-naphthalin-1-on, 8,9,10,11-Tetrahydro-cyclohepta[α]naphthalin-1-on, 3,4-Dihydro-2H-anthracen-1-on oder 6-(4-Chlor-phenyl)-3,4-dihydro-2H-naphthalin-1-on anstelle von 3,4-Dihydro-2H-naphthalin-1-on sowie ggf. anderer Amine in Stufe 1 wurden unter Anwendung der in Beispiel 1 beschriebenen Verfahrensweise analog erhalten:

### 2a : 3-(6-Dimethylaminomethyl-8,9-dihydro-7H-benzocyclohepten-5-yl)-phenol, Hydrochlorid

Schmelzpunkt: 218 - 220°C.

### 2b : 3-(6-Diethylaminomethyl-8,9-dihydro-7H-benzocyclohepten-5-yl)-phenol, Hydrochlorid

Schmelzpunkt: 208 - 211°C

### 2c : 3-(6-Di-n-propylaminomethyl-8,9-dihydro-7H-benzocyclohepten-5-yl)-phenol, Hydrochlorid

Schmelzpunkt: 199 - 201°C

### 2d : 3-{6-[(Methyl-phenethyl-amino)-methyl]-8,9-dihydro-7H-benzocyclohepten-5-yl}-phenol, Hydrochlorid

Schmelzpunkt: Zersetzung ab 117°C

### 2e : 3-{6-[(Benzyl-methyl-amino)-methyl]-8,9-dihydro-7H-benzocyclohepten-5-yl}-phenol, Hydrochlorid

Schmelzpunkt: Zersetzung ab 80°C

### 2f : 3-(6-Dimethylaminomethyl-7,8,9,10-tetrahydrobenzocycloocten-5-yl)-phenol, Hydrochlorid

Schmelzpunkt: 251 - 253,5° C

### 2g : 3-{6-[(Cyclopropylmethyl-methyl-amino)-methyl]-8,9-dihydro-7H-benzocyclohepten-5-yl}-phenol, Hydrochlorid

Schmelzpunkt: 200 - 202° C

### 2h : 3-(6-{[Methyl-(2-pyridin-2-yl-ethyl)-amino]-methyl}-8,9-dihydro-7H-benzocyclohepten-5-yl)-phenol, Dihydrochlorid

Schmelzpunkt: 100 - 105° C

### 2i : 3-(2-Dimethylaminomethyl-3H-inden-1-yl)-phenol, Hydrochlorid

Schmelzpunkt: 210 - 212° C

### 2j : 3-(2-Dimethylaminomethyl-3,4-dihydro-phenanthren-1-yl)-phenol, Hydrochlorid

Schmelzpunkt: 253 - 254° C

### 2k : 3- (2-Dimethylaminomethyl-5-phenyl-3,4-dihydronaphth-1-yl)-phenol, Hydrochlorid

Schmelzpunkt: 250 - 253,5°C

### 2l : 3-(2-Dimethylaminomethyl-6-phenyl-3,4-dihydronaphth-1-yl)-phenol, Hydrochlorid

Schmelzpunkt: 242 - 243°C

### 2m : 3-[6-(3-Chlor-phenyl)-2-dimethylaminomethyl-3,4-dihydro-naphth-1-yl]-phenol, Hydrochlorid

Schmelzpunkt: Zersetzung ab 152°C

### 2n : 3-(8-Dimethylaminomethyl-10,11-dihydro-9H-cyclohepta[α]naphth-7-yl)phenol, Hydrochlorid

Schmelzpunkt: 264 - 267°C

### 2o : 3- (2-Dimethylaminomethyl-3,4-dihydro-anthracen-1-yl)-phenol, Hydrochlorid

Schmelzpunkt: 220 - 222°C

### 2p : 3-[6-(4-Chlor-phenyl)-2-dimethylaminomethyl-3,4-dihydro-naphth-1-yl]-phenol, Hydrochlorid

Schmelzpunkt: 245 - 247°C

### 2q : 2-{6-[(Furan-3-ylmethyl-amino)-methyl]-8,9-dihydro-7H-benzocyclohepten-5-yl}-phenol, Hydrochlorid

### Beispiel 3

### (6-Methoxy-1-phenyl-3,4-dihydro-naphth-2-ylmethyl)-dimethylamin, Hydrochlorid

### 1. Stufe:

Eine Lösung von 50 g 6-Methoxy-3,4-dihydro-2H-naphthalin-1-on in 500 ml Acetonitril wurde mit 26,6 g N,N-Dimethylmethylenimmoniumchlorid und zwei Tropfen Acetylchlorid versetzt und die Mischung 30 Stunden bei 20°C gerührt. Das kristalline Produkt wurde isoliert, mit Adeton gewaschen und im Vakuum bei 40°C getrocknet. Man erhielt so 70,9 g (92,5 %) des Hydrochlorids der Titelverbindung (Schmelzpunkt: 180 - 182°C), aus dem mit verdünnter Natronlauge die Base freigesetzt wurde, die man mit Dichlormethan extrahierte. Nach Trocknen der Extrakte über Natriumsulfat und Abdampfen des Lösungsmittels im Vakuum blieben 56,3 g (RS)-2-Dimethylaminomethyl-6-methoxy-3,4-dihydro-2H-naphthalin-1-on in Form eines gelblichen Öls zurück.

### 2. Stufe:

### (1 RS, 2 RS)-2-Dimethylaminomethyl-6-methoxy-1-phenyl-1,2,3,4-tetrahydro-naphth-1-ol

Eine Lösung von 20,5 g des Produkts aus Stufe 1 in 300 ml trockenem Diethylether wurde bei -60°C unter Rühren und Überleiten von trockenem Stickstoff tropfenweise mit 50 ml einer 2-molaren Lösung von Phenyllithium in Cyclohexan/Diethylether = 70/30 versetzt. Es wurde 2 Stunden bei -60°C nachgerührt und dann mit 150 ml einer gesättigten Ammoniumchloridlösung zersetzt. Die organische Phase wurde abgetrennt, die wässrige noch zweimal mit Essigsäureethylester extrahiert. Die vereinigten organischen Phasen wurden mit einer gesättigten Natriumchloridlösung gewaschen und über Natriumsulfat getrocknet. Das nach Abdampfen der Lösungsmittel im Vakuum zurückbleibende Öl wurde durch Säulenchromatographie mit Diisopropylether als Elutionsmittel gereinigt. Dabei fielen 21,3 g (78,0 % d.Th.) (1 RS, 2 RS)-2-Dimethylaminomethyl-6-methoxy-1-phenyl-1,2,3,4-tetrahydro-naphth-1-ol als Öl an.

### 3. Stufe:

### (6-Methoxy-1-phenyl-3,4-dihydro-naphth-2-ylmethyl)-dimethylamin, Hydrochlorid

Eine Lösung von 6,3 g des Produkts aus Stufe 2 in 100 ml Salzsäure (10 %) wurde 12 Stunden bei 20°C gerührt. Dann wurde mit 1N Natronlauge alkalisiert und dreimal mit Dichlormethan extrahiert. Die Extrakte wurden mit einer gesättigten Natriumchloridlösung gewaschen und über Natriumsulfat getrocknet. Das nach Abdampfen des Lösungsmittels im Vakuum erhaltene Rohprodukt wurde säulenchromatographisch mit Essigsäureethyester/Methanol = 3/1 als Elutionsmittel gereinigt. Die Base wurde dann mit Chlor-trimethylsilan/Wasser in 2-Butanon ins Hydrochlorid übergeführt. (Ausbeute: 5,4 g = 81 % d.Th.; Schmelzpunkt: 189 - 191°C).

### Beispiel 4

### (5-Methoxy-1-phenyl-3,4-dihydro-naphth-2-ylmethyl)-dimethylamin, Hydrochlorid

Bei Verwendung von 5-Methoxy-3,4-dihydro-2H-naphthalin-1-on als Ausgangsverbindung wurde nach der in Beispiel 3 beschriebenen Reaktionsfolge und Verfahrensweise die Titelverbindung in Form weißer Kristalle erhalten. Schmelzpunkt: 205 - 206°C

### Beispiel 5

### 5a : 6-Dimethylaminomethyl-5-phenyl-7,8-dihydro-naphth-2-ol, Hydrochlorid

3,5 g des Produkts aus Beispiel 3 wurden wie in Beispiel 1, Stufe 3, beschrieben mit 100 ml einer Lösung von Bromwasserstoff in Eisessig (33 % HBr) umgesetzt. Nach entsprechender Aufarbeitung, säulenchromatographischer Reinigung und Umsetzung mit Trimethylchlorsilan/Wasser wurden 2,4 g (63,7 % d.Th.)der Titelverbindung in Form weißer Kristalle erhalten.
Schmelzpunkt: 189 - 191°C

### 5b : 6-Dimethylaminomethyl-5-phenyl-7,8-dihydro-naphth-1-ol, Hydrochlorid

Unter Anwendung der in Beispiel 5 a beschriebenen Verfahrensweise erhielt man aus dem Produkt aus Beispiel 4 analog die Titelverbindung.
Schmelzpunkt: 245 - 247°C

### Beispiel 6

### 6-Dimethylaminomethyl-5-(3-hydroxy-phenyl)-7,8-dihydronaphth-2-ol, Hydrochlorid

### 1. Stufe:

### (1RS, 2RS)-2-Dimethylaminomethyl-6-methoxy-1-(3-methoxy-phenyl)-1,2,3,4-tetrahydro-naphth-1-ol

Aus 7,3 g Magnesiumspänen und 56,1 g 1-Brom-3-methoxybenzol in 200 ml trockenem Tetrahydrofuran wurde unter leichtem Sieden das entsprechende Grignardreagenz dargestellt. Dazu tropfte man bei +5 bis 10°C eine Lösung von 46,7 g (RS)-2-Dimethylaminomethyl-6-methoxy-3,4-dihydro-2H-naphthalin-1-on (Produkt aus Beispiel 3, 1. Stufe) in 100 ml trockenem Tetrahydrofuran. Es wurde 16 Stunden bei + 22°C nachgerührt und, nach Abkühlen auf ca. 10°C, mit 100 ml einer gesättigten Ammoniumchloridlösung zersetzt. Die Reaktionsmischung verdünnte man mit 100 ml Wasser und 200 ml Diethylether, trennte die Phasen und extrahierte die wäßrige Phase noch zweimal mit je 100 ml Diethylether. Die vereinigten organischen Phasen wurden über Natriumsulfat getrocknet und im Vakuum von den flüchtigen Bestandteilen befreit. Der ölige Rückstand wurde durch Säulenchromatographie mit Essigsäureethylester als Elutionsmittel gereinigt, wobei 43,9 g (64,3 % d.Th.) (1RS, 2RS)-2-Dimethylaminomethyl-6-methoxy-1-(3-methoxy-phenyl)-1,2,3,4-tetrahydronaphth-1-ol anfielen.

### 2. Stufe:

### 6-Dimethylaminomethyl-5-(3-hydroxy-phenyl)-7,8-dihydronaphth-2-ol, Hydrochlorid

34,2 g des Produkts aus Stufe 1 wurden mit 350 ml einer Lösung von Bromwasserstoff in Eisessig (33 % HBr) 20 Stunden bei einer Temperatur von 100° bis 110° C gerührt. Dann wurde im Vakuum eingedampft und der Rückstand in 500 ml Wasser aufgenommen. Nach Aufarbeitung wie in Beispiel 1, Stufe 2, beschrieben, reinigte man säulenchromatographisch mit Essigsäureethylester/Methanol = 3/1 als Elutionsmittel. Die so erhaltene Base der Titelverbindung wurde mit Trimethylchlorsilan/Wasser in 2-Butanon ins Hydrochlorid übergeführt.
Ausbeute: 12,2 g (41,2 % d.Th.)
Schmelzpunkt: 210 - 212°C

### Beispiel 7

Die zu dem Produkt in Beispiel 6 isomeren Verbindungen 7a und 7b wurden durch Verwendung entsprechender Ausgangssubstanzen nach der in Beispiel 6 beschriebenen Verfahrensweise erhalten:

### 7a : 6-Dimethylaminomethyl-5-(3-hydroxy-phenyl)-7,8-dihydro-naphth-1-ol, Hydrochlorid

Schmelzpunkt: 260 - 262°C

### 7b : 7-Dimethylaminomethyl-8-(3-hydroxy-phenyl)-5,6-dihydro-naphth-2-ol, Hydrochlorid

Schmelzpunkt: 239 - 242°C

Ersatz von (RS)-2-Dimethylaminomethyl-6-methoxy-3,4-dihydro-2H-naphthalin-1-on in Beispiel 6, Stufe 1, durch (RS)-6-Dimethylaminomethyl-2-methoxy-6,7,8,9-tetrahydro-benzocyclohepten-5-on (7c) bzw. (RS)-2-Dimethylaminomethyl-6-(3-methoxyphenyl)-3,4-dihydro-2H-naphthalin-1-on (7d) und Weiterreaktion nach der in Beispiel 6 beschriebenen Verfahrensweise lieferte:

### 7c : 6-Dimethylaminomethyl-5-(3-hydroxy-phenyl)-8,9-dihydro-7H-benzocyclohepten-2-ol, Hydrochlorid

Schmelzpunkt: Zersetzung ab 110°C

### 7d : 3- [2-Dimethylaminomethyl-6-(3-hydroxy-phenyl)-3,4-dihydro-naphth-1-yl]-phenol, Hydrochlorid

### Beispiel 8

### 3-(2-Dimethylaminomethyl-7-phenoxy-3,4-dihydro-naphth-1-yl)-phenol, Hydrochlorid

### 1. Stufe:

### 7-Phenoxy-3,4-dihydro-2H-naphthalin-1-on

Eine Suspension von 60,8 g Kaliumcarbonat in 300 ml trockenem Pyridin wurde mit 35,7 g 7-Hydroxy-3,4-dihydro-2H-naphthalin-1-on versetzt und die Mischung auf 40°C erwärmt. Danach gab man unter Rühren zunächst 19,9 g Kupfer(II)-oxid zu, anschließend tropfenweise 39,6 g Brombenzol. Das Reaktionsgemisch wurde 4 Tage zum Rückfluß erhitzt. Nach gründlichem Abdampfen des Pyridins im Vakuum verrührte man den Rückstand mit 200 ml Essigsäureethylester und filtrierte über Kieselgel. Das Filtrat wurde mit gesättigten Lösungen von Ammoniumchlorid und Natriumchlorid gewaschen, über Natriumsulfat getrocknet und im Vakuum eingedampft. Den Rückstand reinigte man säulenchromatographisch mit n-Hexan/Essigsäureethylester als Elutionsmittel und erhielt dabei 41,2 g (78,6 % d. Th.) 7-Phenoxy-3,4-dihydro-2H-naphthalin-1-on.

### 2. Stufe:

### 2-Dimethylaminomethyl-7-phenoxy-3,4-dihydro-2H-naphthalin-1-on

Analog Beispiel 3, Stufe 1, wurden 40,5 g des Produkts aus Stufe 1 in 500 ml Acetonitril mit 16,0 g N,N-Dimethylmethylen-immoniumchlorid umgesetzt. Man erhielt nach entsprechender Aufarbeitung 45,8 g (91,3 % d. Th.) 2-Dimethylaminomethyl-7-phenoxy-3,4-dihydro-2H-naphthalin-1-on in Form eines Öls.

### 3. Stufe:

### (1RS, 2RS)-1-[3-(tert-Butyl-diphenyl-silanyloxy)-phenyl]-2-dimethylaminomethyl-7-phenoxy-1,2,3,4-tetrahydro-naphth-1-ol

Eine Lösung von 41,2 g (3-Brom-phenoxy)-tert-butyl-diphenyl-silan in 300 ml trockenem Tetrahydrofuran wurde bei -40°C unter Rühren und Überleiten von trockenem Stickstoff tropfenweise mit 62,5 ml einer 1,6 M Lösung von n-Butyllithium in n-Hexan versetzt. Man rührte 30 Minuten nach und tropfte dann eine Lösung von 25,1 g des Produkts aus Stufe 2 in 75 ml trockenem Tetrahydrofuran zu. Das Reaktionsgemisch ließ man innerhalb von 12 Stunden auf +20°C erwärmen und zersetzte durch Zugabe von 100 ml einer gesättigten Ammoniumchloridlösung. Nach Verdünnen mit je 200 ml Wasser und Essigsäurèethylester wurde die organische Phase abgetrennt und die wäßrige Phase zweimal mit 100 ml Essigsäureethylester extrahiert. Die vereinigten organischen Phasen wurden mit einer gesättigten Natriumchloridlösung gewaschen, über Natriumsulfat getrocknet und im Vakuum eingedampft. Man reinigte den Rückstand säulenchromatographisch mit Essigsäureethylester als Elutionsmittel und erhielt so 32,9 g (61,6 % d.Th.) (1RS, 2RS)-1-[3-(tert-Butyl-diphenyl-silanyloxy)-phenyl]-2-dimethylaminomethyl-7-phenoxy-1,2,3,4-tetrahydro-naphth-1-ol als nahezu farbloses viskoses Öl.

### 4. Stufe:

### (1RS, 2RS)-2-Dimethylaminomethyl-1-(3-hydroxy-phenyl)-7-phenoxy-1,2,3,4-tetrahydro-naphth-1-ol

Eine Lösung von 31,4 g des Produkts aus Stufe 3 in 360 ml trockenem Tetrahydrofuran wurde bei +5°C bis 10°C unter Rühren tropfenweise mit 57,5 ml einer 1-molaren Lösung von Tetra-n-butylammoniumfluorid in Tetrahydrofuran versetzt. Nach beendeter Zugabe rührte man 3 Stunden bei 20°C, versetzte mit 150 ml einer gesättigten Lösung von Natriumchlorid und extrahierte dreimal mit je 150 ml Essigsäureethylester. Die Extrakte wurden über Natriumsulfat getrocknet und im Vakuum eingedampft. Man reinigte den Rückstand säulenchromatographisch mit Essisäureethylester/Methanol = 5/1 als Elutionsmittel und erhielt so 17,3 g (88,7 % d. Th.) (1RS, 2RS)-2-Dimethylaminomethyl-1-(3-hydroxy-phenyl)-7-phenoxy-1,2,3,4-tetrahydro-naphth-1-ol als leicht gelb gefärbtes viskoses Öl.

### 5. Stufe:

### 3-(2-Dimethylaminomethyl-7-phenoxy-3,4-dihydro-naphth-1-yl)-phenol, Hydrochlorid

15,6 g des Produkts aus Stufe 4 wurden wie in Beispiel 3,' Stufe 3, beschrieben, mit 150 ml einer 6 N Salzsäure zur Reaktion gebracht. Nach analoger Aufarbeitung und Überführung ins Hydrochlorid erhielt man 12,1 g (73,8 % d. Th.) 3-(2-Dimethylaminomethyl-7-phenoxy-3,4-dihydronaphth-1-yl)-phenol, Hydrochlorid in Form weißer Kristalle.
Schmelzpunkt: 210 - 212°C

### Beispiel 9

In Beispiel 8, Stufe 2, wurde 7-Phenoxy-3,4-dihydro-2Hnaphthalin-1-on durch die entsprechenden 5-Methoxy-(9a), 7-Phenyl- (9b), 7-Phenethyl- (9d), 5-Phenoxy-(9e), 6-Phenoxy- (9f) und 7-n-Butylderivate (9c) bzw. durch 2-Phenoxy-6,7,8,9-tetrahydro-benzocyclohepten-5-on (9g) ersetzt. In einer Beispiel 8 analogen Reaktionsfolge und Verfahrensweise erhielt man daraus:

### 9a : 3- (2-Dimethylaminomethyl-5-methoxy-3,4-dihydronaphth-1-yl)-phenol, Hydrochlorid

Schmelzpunkt: 245 - 247°C

### 9b : 3-(2-Dimethylaminomethyl-7-phenyl-3,4-dihydronaphth-1-yl)-phenol, Hydrochlorid

Schmelzpunkt: 232 - 233°C

### 9c : 3- (7-n-Butyl-2-dimethylaminomethyl-3,4-dihydronaphth-1-yl)-phenol, Hydrochlorid

Schmelzpunkt: 202 - 205°C

### 9d : 3-(2-Dimethylaminomethyl-7-phenethyl-3,4-dihydronaphth-1-yl)-phenol, Hydrochlorid

Schmelzpunkt: 233 - 237°C

### 9e : 3-(2-Dimethylaminomethyl-5-phenoxy-3,4-dihydronaphth-1-yl)-phenol, Hydrochlorid

Schmelzpunkt: 197 - 198°C

### 9f : 3-(2-Dimethylaminomethyl-6-phenoxy-3,4-dihydronaphth-1-yl)-phenol, Hydrochlorid

Schmelzpunkt: 224,5 - 226°C

### 9g 3- (6-Dimethylaminomethyl-2-phenoxy-8,9-dihydro-7Hbenzocyclohepten-5-yl)-phenol, Hydrochlorid

Schmelzpunkt: 245 - 247°C

### Bespiel 10

### 3-{6-[Allyl-methyl-amino)-methyl]-8,9-dihydro-7H-benzocyclohepten-5-yl}-phenol, Hydrochlorid

### 1. Stufe:

### 6-[(Allyl-methyl-amino)-methyl]-6,7,8,9-tetrahydro-benzocyclohepten-5-on

5,2 g 6,7,8,9-Tetrahydro-benzocyclohepten-5-on, 0,96 g Paraformaldehyd und 10,0 g Allyl-methyl-amin, Hydrochlorid in 60 ml Eisessig wurden wie in Beispiel 1, Stufe 1, zur Reaktion gebracht. Nach analoger Aufarbeitung erhielt man 6,7 g (84,8 % d. Th.) 6-[(Allylmethyl-amino)-methyl]-6,7,8,9-tetrahydro-benzocyclohepten-5-on als gelbliches Öl.

### Endstufe:

### 3-{6-[Allyl-methyl-amino)-methyl]-8, 9-dihydro-7H-benzocyclohepten-5-yl}-phenol, Hydrochlorid

Das Produkt aus Stufe 1 wurde nach der in Beispiel 8, Stufen 3 - 5, beschriebenen Verfahrensweise weiter umgesetzt. Man erhielt so 3-{6-[Allyl-methyl-amino)-methyl]-8,9-dihydro-7H-benzocyclohepten-5-yl}-phenol, Hydrochlorid in Form weißer Kristalle.
Schmelzpunkt: 156 - 159°C (Zers.)

### Beispiel 11

### a) 3- [3-Dimethylamino-1-(3-hydroxy-phenyl)-2-methylpropenyl]-phenol, Hydrochlorid

### 1. Stufe:

### (2RS)-3-Dimethylamino-1,1-bis- (3-methoxy-phenyl)-2-methyl-propan-1-ol, Hydrochlorid

27,0 g Magnesiumspäne wurden in 150 ml trockenem Tetrahydrofuran gerührt. Man tropfte 207,6 g 1-Brom-3-methoxy-benzol, gelöst in 400 ml trockenem Tetrahydrofuran, so zu, daß das Reaktionsgemisch leicht siedete. Nach der Zugabe erhitzte man noch eine Stunde unter Rückfluß, kühlte dann auf +5 bis 10°C ab und tropfte bei dieser Temperatur 166,0 g (RS)-3-Dimethylamino-1-(3-methoxy-phenyl)-2-methyl-propan-1-on, gelöst in 400 ml Tetrahydrofuran, zu. Das Reaktionsgemisch wurde über Nacht stehengelassen und anschließend erneut auf 5 bis 10° C abgekühlt. Durch Zugabe von 300 ml 20 %iger Ammoniumchloridlösung wurde die Grignardlösung zersetzt. Die Reaktionsmischung verdünnte man mit 400 ml Ether, trennte die Phasen und extrahierte die wäßrige Phase zweimal mit 250 ml Ether. Die vereinigten organischen Phasen wurden über Natriumsulfat getrocknet und vom Lösungsmittel befreit. Der Rückstand (342 g) wurde in 4000 ml 2-Butanon aufgenommen und mit 81,5 g Trimethylchlorsilan und 13,5 ml Wasser versetzt. Über Nacht kristallisierten bei 4 bis 5° C 165,0 g (60 % d. Th.) 3,3-Bis-(2-hydroxy-phenyl-2-methyl-prop-2-enyl)-dimethylamin, Hydrochlorid aus.

Schmelzpunkt: 158 - 160°C

### 2. Stufe:

### 3-[3-Dimethylamino-1-(3-hydroxy-phenyl)-2-methyl-propenyl]-phenol, Hydrochlorid

58 g (2RS)-3-Dimethylamino-1,1-bis-(3-methoxy-phenyl)-2-methyl-propan-1-ol, Hydrochlorid aus Stufe 1 wurden in 2000 ml konzentrierter Bromwasserstoffsäure (47 %ig) gelöst und 7 Stunden unter Rückfluß erhitzt. Nach Abkühlung auf Raumtemperatur wurde das Reaktionsgemisch mit 800 ml Wasser, 2000 ml Dichlormethan und 80 g Natriumhydrogencarbonat versetzt. Nach Abtrennung der Dichlormethanphase wurde die Wasserphase zweimal mit 1000 ml Essigsäureethylester extrahiert. Die vereinigten organischen Phasen wurden über Natriumsulfat getrocknet und vom Lösungsmittel befreit. Der Rückstand (42 g), gelöst in einem Gemisch bestehend aus 100 ml Essigsäureethylester und 100 ml Tetrahydrofuran, wurde durch Säulenchromatographie gereinigt. Die Elution mit Essigsäureethylester/Methanol = 4/1 ergab 22 g Base. Diese wurden in 500 ml 2-Butanon gelöst und mit 8,5 g Trimethylchlorsilan und 16 ml Wasser versetzt. Das auskristallisierte Hydrochlorid (14,6 g) wurde abgesaugt und zur Reinigung in 1000 ml 2-Butanon suspendiert. Die Suspension wurde 5 Stunden unter Rückfluß gerührt. Nach Abkühlung auf Raumtemperatur wurden 13,5 g Hydrochlorid (26,4 d. Th.) erhalten.

Schmelzpunkt: 222 - 224°C

### b), Z-3-(1-Biphenyl-4-yl-3-dimethylamino-2-methyl-propenyl)-phenol, Hydrochlorid

Nach der in Beispiel 11a beschriebenen Verfahrensweise und bei Verwendung von (RS)-1-Biphenyl-4-yl-3-dimethylamino-2-methyl-propan-1-on erhielt man analog die Titelverbindung.

Schmelzpunkt: 192 - 194° C

Unter Verwendung entsprechender substituierter Dimethylamino-alkan-1-one in Stufe 1 erhielt man nach analoger Reaktionsfolge:

### 11c : E-3-[1-(3,4-Dichlor-phenyl)-3-dimethylamino-2-methyl-propenyl]-phenol, Hydrochlorid

Schmelzpunkt: 176 - 178°C

### 11d : Z-3-[1-(4-Chlor-phenyl)-3-dimethylamino-2-methylpropenyl]-phenol, Hydrochlorid

Schmelzpunkt: 144 - 146°C

### 11e : Z-3-[3-Dimethylamino-2-methyl-1-(4-phenoxyphenyl)-propenyl]-phenol, Hydrochlorid

Schmelzpunkt: 190 - 192°C

### 11f : Z-3-(3-Dimethylamino-2-methyl-1-p-tolyl-propenyl)-phenol, Hydrochlorid

Schmelzpunkt: 200 - 201°C

### 11g : Z-3-(2-Dimethylaminomethyl-1-phenyl-but-1-enyl)-phenol, Hydrochlorid

Schmelzpunkt: 188 - 190°C

### 11h : Z-3-[1-(4'-Chlor-biphenyl-4-yl)-3-dimethylamino-2-methyl-propenyl]-phenol, Hydrochlorid

Schmelzpunkt: 156 - 158°C

### 11i : Z-3-[3-Dimethylamino-2-methyl-1-(4-styryl-phenyl)-propenyl]-phenol, Hydrochlorid

Schmelzpunkt: 236 - 237°C

### 11j : Z-3-[3-Dimethylamino-2-methyl-1-(4-phenethylphenyl)-propenyl]-phenol, Hydrochlorid

Schmelzpunkt: 183 - 185°C

### 11k : Z-3-[3-Dimethylamino-1-(4-hydroxy-phenyl)-2-methyl-propenyll-phenol, Hydrochlorid

### 11l : Z-3-(1-Benzo[1,3]dioxol-5-yl-3-dimethylamino-2-methyl-propenyl)-phenol, Hydrochlorid

Schmelzpunkt: 121 - 124°C

Die unten beschriebenen Delta-Opiatrezeptor-Bindungsuntersuchungen zeigen, daß die erfindungsgemäßen Verbindungen der Formel (I) hervorragende Wirkungen als Analgetika aufweisen.

### δ-Opiatrezeptor-Bindungsuntersuchungen

Die Untersuchungen zur Bestimmung der Affinität der erfindungsgemäßen Verbindungen der Formel I zum δ-Opiatrezeptor wurde an Hirnmembranhomogenaten (Homogenat von Rattenhirn ohne Cerebellum, Pons und Medulla oblongata von männlichen Wistar-Ratten) durchgeführt.

Hierzu wurde das jeweils frisch präparierte Rattenhirn unter Eiskühlung in 50 mmol/l Tris-HCl (pH 7,4) homogenisiert und für 10 Minuten bei 5.000 g und 4°C zentrifugiert. Nach Dekantieren und Verwerfen des Überstandes, erneutem Aufnehmen und Homogenisieren des Membransedimentes in 50 mmol/l Tris-HCl (pH 7,4) wurde das Homogenat anschließend für 20 Minuten bei 20.000 g und 4°C zentrifugiert. Dieser Waschschritt wurde nochmals wiederholt. Danach wurde der Überstand dekantiert und das Membransediment in kaltem 50 mmol/l Tris-HCl, 20 % Glycerol (w/v), 0,01 % Bacitracin (w/v) (pH 7,4) homogenisiert und in Aliquoten bis zur Testung eingefroren. Für die Rezeptorbindungstestung wurden die Aliquote aufgetaut und 1:10 mit dem Bindungstest-Puffer verdünnt.

Im Bindungstest wurde als Puffer ein 50 mmol/l Tris-HCl, 5 mmol/l MgCl₂ (pH 7,4) supplementiert mit 0,1 % (w/v) bovinem Serumalbumin, sowie als radioaktiver Ligand 1 nmol/l (³H)-2-D-Ala-Deltorphin II eingesetzt. Der Anteil an unspezifischer Bindung wurde in Anwesenheit von 10 µmol/l Naloxon bestimmt.

In weiteren Ansätzen wurden die erfindungsgemäßen Verbindungen in Konzentrationsreihen zugegeben und die Verdrängung des radioaktiven Liganden aus seiner spezifischen Bindung ermittelt. Die jeweiligen Dreifachansätze wurden über 90 Minuten bei 37° C inkubiert und anschließend zur Bestimmung des an das Membranhomogenat gebundenen radioaktiven Liganden mittels Filtration durch Glasfaserfilter (GF/B) geerntet. Die Radioaktivität der Glasfaserfilterscheiben wurde nach Zugabe von Szintillator im β-Counter gemessen.

Die Affinität der erfindungsgemäßen Verbindungen zum δ-Opiatrezeptor wurde als IC₅₀ nach dem Massenwirkungsgesetz mittels nichtlinearer Regression berechnet. Aus den IC₅₀-Werten wurden nach der Cheng-Prussoff-Gleichung Kᵢ-Werte berechnet. Die Kᵢ-Werte sind als Mittelwerte ± Standardabweichungen von ≥ 3 voneinander unabhängigen Versuchen angegeben.

**Tabelle 1**

| **Bsp.- Nr.** | **δ-Opiatrezeptorbindung K**_{**i**} **(nmol/l)** |
|---|---|
| 1 | 55,8 ± 4,6 |
| 2a | 17,2 ± 4,3 |
| 2b | 728,0 ± 146,0 |
| 2c | 294,0 ± 106,0 |
| 2d | 54,3 ± 8,2 |
| 2e | 936,0 ± 32,0 |
| 2f | 46,3 ± 10,1 |
| 2g | 182,0 ± 12,0 |
| 2h | 223,0 ± 86,0 |
| 2i | 157,0 ± 16,0 |
| 2j | 80,8 ± 29,7 |
| 3 | 456,0 ± 62,0 |
| 4 | 517,0 ± 45, 0 |
| 5 | 843,0 ± 23,0 |
| 6 | 40,9 ± 6,7 |
| 7 | 21,6 ± 6,2 |
| 8 | 25,7 ± 10,2 |
| 9a | 23,9 ± 0,9 |
| 9b | 39,3 ± 2,2 |
| 9c | 42,2 ± 16,0 |
| 10 | 679,0 ± 214,0 |
| 11a | 34,0 ± 5,8 |
| 11b | 23,7 ± 1,7 |

Tabelle 1 zeigt, daß die erfindungsgemäßen Aminoverbindungen analgetisch wirksam sind, wobei diese biologische Wirksamkeit teilweise oder überwiegend über δ-Opiatrezeptoren vermittelt und dadurch nachgewiesen wird.

Als wirksame und selektive δ-Opioid-Agonisten und -Antagonisten können die erfindungsgemäßen Verbindungen der allgemeinen Formel (I), als Mittel bei pathologischen Zuständen, die üblicherweise mit Agonisten und Antagonisten des δ-Opioid-Rezeptors behandelt werden, eingesetzt werden. Vorzugsweise können die Verbindungen der allgemeinen Formel I als Analgetika verwendet werden.

## Patentansprüche

1. Substituierte Aminoverbindungen der allgemeinen Formel I, 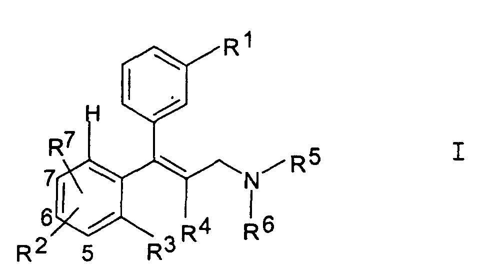 worin
R¹ H, OH, O-C₁₋₆-Alkyl, O-C₃₋₇-Cycloalkyl; (wobei: R¹ ungleich H, wenn R² und R⁷ gleich H oder in 7-Stellung eine OCH₃-Gruppe substituiert ist) ;
R² H, OH, C₁₋₆-Alkyl, Aryl(wobei Aryl unsustituierte oder ein- oder mehrfach mit OH, F, Cl, CF₃, C₁₋₆-Alkyl, C₁₋₆-Alkoxy, Cycloalkoxy mit bis zu 7 C-Atomen, C₃₋₇-Cycloalkyl, C₂₋₆-Alkylen oder Phenyl substituierte Phenyle bedeutet, wobei wiederum die Phenylreste ankondensiert sein können, oder Aryl auch Naphthyl bedeutet), O-Aryl, C₂₋₆-Alkenylen-Aryl, O-C₃₋₇-Cycloalkyl, C1, F, C₁₋₆-Alkoxy, C₁₋₆-Alkyl-Aryl, 5,6- oder 6,7-Benzo-(an)kondensiert unsubstituiert, mono- oder disubstituiert mit Cl, F, CF₃, C₁₋₆-Alkyl, O-C₁-6-Alkyl, OH,
(wobei: wenn R¹ und R² oder R⁷ gleich H ist, kann R² oder R⁷ keine CF₃-Gruppe und R² oder R⁷ in 7-Stellung keine OCH₃-Gruppe darstellen) oder R² und R⁷ zusammen -O-(CH₂)₍₁₋₂₎-O- (in 5,6- oder 6,7-Position);
R³ H;
R⁴ C₁₋₆-Alkyl,
oder R³ und R⁴ zusammen -(CH₂)₍₁₋₄₎-;
R⁵ C₁₋₆-Alkyl, C₃₋₇-Cycloalkyl;
R⁶ C₁₋₆-Alkyl, C₁₋₆-Alkyl-Aryl; C₁₋₆-Alkyl-Heterocyclyl, wobei Heterocyclyl Furan, Thiophen, Pyridin, Pyrimidin, Thiazol, Oxazol, Isoxazol, Pyridazin, Pyrazin, Chinolin, Isochinolin, Phthalazin oder Chinazolin bedeutet, -CH₂-CH=C(R⁸)₂, -CH₂-(C₃₋₇)-Cycloalkyl, C₃₋₇-Cycloalkyl
R⁷ H, OH, C₁₋₆-Alkyl, O-Aryl, C₂₋₆-Alkenylen-Aryl, O-C₃₋₇-Cycloalkyl, C₁₋₆-Alkoxy, Cl, F, Aryl, C₁₋₆-Alkyl-Aryl, 5,6- oder 6,7-Benzo-(an)kondensiert unsubstituiert, monooder disubstituiert mit Cl, F, CF₃, C₁₋₆-Alkyl, O-C₁₋₆-Alkyl, OH,
(wobei: wenn R¹ und R² oder R⁷ gleich H ist,kann R² oder R⁷ keine CF₃-Gruppe und R² oder R⁷ in 7Stellung keine OCH₃-Gruppe darstellen), und
R⁸ H oder CH₃
bedeuten oder pharmazeutisch verwendbare Salze davon.

2. Substituierte Aminoverbindungen gemäß Anspruch 1, **dadurch gekennzeichnet, daß** R⁶ C₁₋₆-Alkyl-Heterocyclyl, wobei Heterocyclyl Furan, Thiophen, Pyridin, Pyrimidin, Thiazol, Oxazol, Isoxazol, Pyridazin, Pyrazin, Chinolin, Isochinolin, Phthalazin oder Chinazolin bedeutet, oder C₁₋₆-Alkyl-Aryl und R² und R⁷ C₁₋₆-Alkyl-Aryl, R¹, R³ bis R⁵ und R⁸ die Bedeutung gemäß Anspruch 1 haben.

3. Substituierte Aminoverbindungen gemäß Anspruch 1, **dadurch gekennzeichnet, daß** R², R⁷ Aryl, R¹, R³ bis R⁶ und R⁸ die Bedeutung gemäß Anspruchs 1 haben.

4. Substituierte Aminoverbindungen gemäß Anspruch 1, **dadurch gekennzeichnet, daß** R¹ OH oder -O-C₁₋₆-Alkyl, R⁵ C₁₋₆-Alkyl, R⁶ C₁₋₆-Alkyl, R² bis R⁴ und R⁷ die Bedeutung gemäß'Anspruchs 1 haben.

5. Substituierte Aminoverbindungen gemäß Anspruch 1, **dadurch gekennzeichnet, daß** R¹ OH, R⁵ Methyl, R⁶ Methyl, R² bis R⁴ und R⁷ die Bedeutung gemäß Anspruch 1 haben.

6. Arzneimittel enthaltend als Wirkstoff wenigstens eine Verbindung gemäß den Ansprüchen 1 bis 5 und gegebenenfalls weitere Wirkstoffe.

7. Arzneimittel nach Anspruch 6 mit analgetischer Wirkung.

8. Verfahren zur Herstellung einer Verbindung der Formel (I) 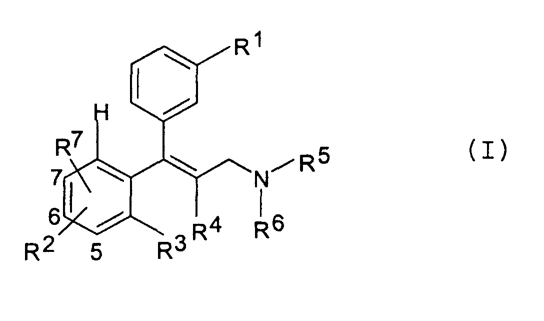 worin R¹ bis R⁷ die Bedeutung gemäß Anspruch 1 haben, **dadurch gekennzeichnet, daß** ein tertiärer Alkohol der allgemeinen Formel (II) 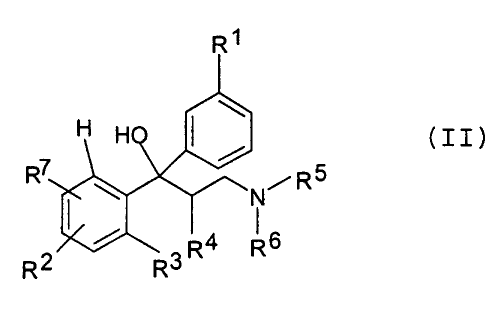 worin R¹ bis R⁷ dieselbe Bedeutung wie in Formel (I) haben, in organischen oder anorganischen Säuren, in einem Temperaturbereich zwischen 0°C und 100° C umgesetzt wird,
wobei der tertiäre Alkohol der allgemeinen Formel (II) dadurch erhalten wird, daß zunächst β-Aminoketone der allgemeinen Formel (III) 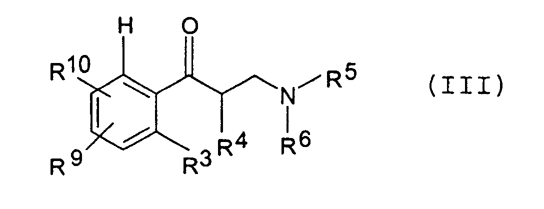 worin R³ bis R⁶ dieselbe Bedeutung wie in Formel(I) haben, R⁹ wie R², und R¹⁰ wie R⁷ definiert ist, mit der Ausnahme, daß die Hydroxy-Funktion in geschützter Form als Benzyloxy- oder Silanyloxygruppe vorliegt, mit einer metallorga-nischen Verbindung der Formel IV, 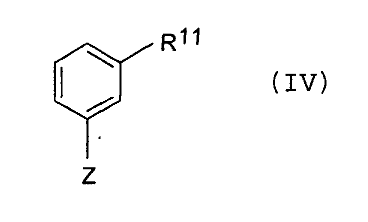 in der Z für MgCl, MgBr, MgI oder Li steht und R¹¹ Wasserstoff, O-C₁₋₆-Alkyl, O-C₃₋₇-Cycloalkyl oder eine geschützte Hydroxy-Funktion darstellt, die als Benzyloxy- oder Silanyloxygruppe vorliegt, die zu einer Verbindung der Formel (IIa) 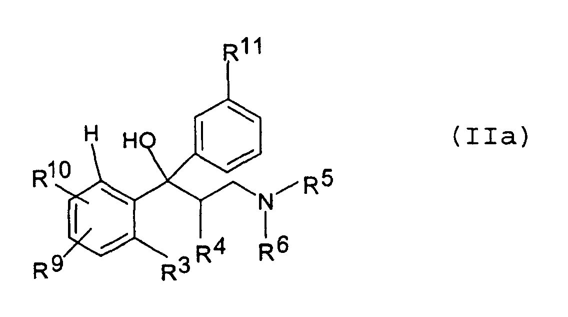 umgesetzt werden und diese dann in die Verbindung der Formel (II) überführt.

9. Verwendung der substituierten Aminoverbindungen der allgemeinen Formel (I) nach Anspruch 1 als Wirkstoff in einem Arzneimittel.

10. Verwendung nach Anspruch 7, **dadurch gekennzeichnet, daß** das Arzneimittel ein Analgetikum ist.

## Claims

1. Substituted amino compounds of the general formula I 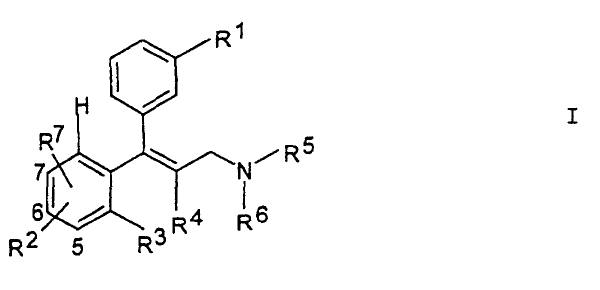 wherein
R¹ denotes H, OH, O-C₁₋₆-alkyl, O-C₃₋₇-cycloalkyl; (wherein: R¹ is not identical to H if R² and R⁷ are identical to H, or an OCH₃ group is substituted in the 7-position);
R² denotes H, OH, C₁₋₆-alkyl, aryl (wherein aryl denotes unsubstituted phenyls or phenyls substituted singly or multiply with OH, F, Cl, CF₃, C₁₋₆-alkyl, C₁₋₆-alkoxy, cycloalkoxy with up to 7 C atoms, C₃₋₇-cyclo-alkyl, C₂₋₆-alkylene or phenyl, wherein in turn the phenyl radicals may be condensed on, or denotes aryl or naphthyl), O-aryl, C₂₋₆-alkenylene-aryl, O-C₃₋₇-cycloalkyl, Cl, F, C₁₋₆-alkoxy, C₁₋₆-alkyl-aryl, 5,6- or 6,7-benzocondensed (on) unsubstituted, monosubstituted or disubstituted with Cl, F, CF₃, C₁₋₆-alkyl, O-C₁₋₆-alkyl, OH, (wherein: if R¹ and R² or R⁷ are identical to H, then R² or R⁷ may not denote a CF₃ group, and R² or R⁷ in the 7-position may not denote an OCH₃ group),
or R² and R⁷ together denote -O-(CH₂)₍₁₋₂₎-O- (in the 5,6- or 6,7-position);
R³ denotes H;
R⁴ denotes C₁-C₆-alkyl, or R³ and R⁴ together denote-(CH₂)₍₁₋₄₎-;
R⁵ denotes C₁-C₆-alkyl, C₃₋₇-cycloalkyl;
R⁶ denotes C₁-C₆-alkyl, C₁-C₆-alkyl-aryl; C₁-C₆-alkylheterocyclyl, wherein heterocyclyl denotes furan, thiophene, pyridine, pyrimidine, thiazole, oxazole, isoxazole, pyridazine, pyrazine, quinoline, isoquinoline, phthalazine or quinazoline, -CH₂-CH=C(R⁸)₂, -CH₂-(C₃₋₇)-cycloalkyl, C₃₋₇-cycloalkyl;
R⁷ denotes H, OH, C₁-C₆-alkyl, O-aryl, C₂₋₆-alkenylene-aryl, O-C₃₋₇-cyclo-alkyl, C₁-C₆-alkoxy, Cl, F, aryl, C₁-C₆-alkyl-aryl, 5,6- or 6,7-benzocondensed (condensed on) unsubstituted, monosubstituted or disubstituted with Cl, F, CF₃, C₁-C₆-alkyl, O-C₁-C₆-alkyl, OH,
(wherein: if R¹ and R² or R⁷ are identical to H, then R² or R⁷ may not denote a CF₃ group and R² or R⁷ in the 7-position may not denote an OCH₃ group), and
R⁸ denotes H or CH₃
or pharmaceutically usable salts thereof.

2. Substituted amino compounds according to claim 1, **characterised in that** R⁶ denotes C₁-C₆-alkylheterocyclyl, wherein heterocyclyl denotes furan, thiophene, pyridine, pyrimidine, thiazole, oxazole, isoxazole, pyridazine, pyrazine, quinoline, isoquinoline, phthalazine or quinazoline, or denotes C₁-C₆-alkyl-aryl, and R² and R⁷ denote C₁-C₆-alkyl-aryl and R¹, R³ to R⁵ and R⁸ have the meanings according to claim 1.

3. Substituted amino compounds according to claim 1, **characterised in that** R², R⁷ denote aryl, and R¹, R³ to R⁶ and R⁸ have the meanings according to claim 1.

4. Substituted amino compounds according to claim 1, **characterised in that** R¹ denotes OH or -O-C₁-C₆-alkyl, R⁵ denotes C₁-C₆-alkyl R⁶ denotes C₁-C₆-alkyl, and R² to R⁴ and R⁷ have the meanings according to claim 1.

5. Substituted amino compounds according to claim 1, **characterised in that** R¹ denotes OH, R⁵ denotes methyl, R⁶ denotes methyl, and R² to R⁴ and R⁷ have the meanings according to claim 1.

6. Medicament containing as active constituent at least one compound according to claims 1 to 5 and optionally further active constituents.

7. Medicament according to claim 6 having an analgesic effect.

8. Process for the production of a compound of the formula (I) 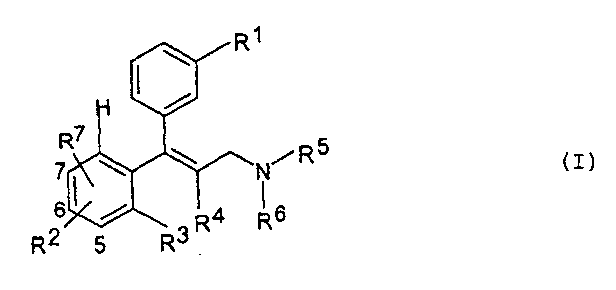 wherein R¹ to R⁷ have the meanings according to claim 1, **characterised in that** a tertiary alcohol of the general formula (II) 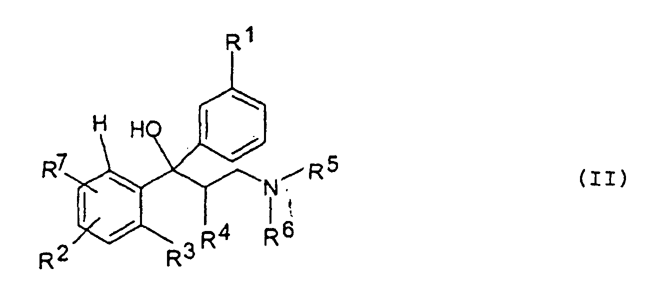 wherein R¹ to R⁷ have the same meanings as in formula (I) is reacted in organic or inorganic acids in a temperature range between 0°C and 100°C, the tertiary alcohol of the general formula (II) thereby being obtained, that first of all β-aminoketones of the general formula (III) 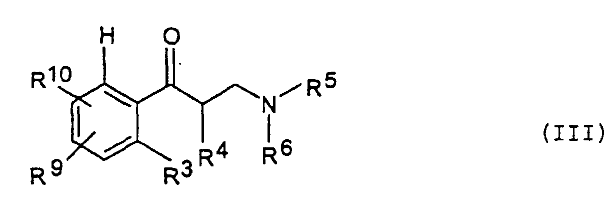 wherein R³ to R⁶ have the same meanings as in formula (I), R⁹ is defined as R² and R¹⁰ is defined as R⁷, except that the hydroxy group is present in protected form as a benzyloxy group or silanyloxy group, are reacted with an organometallic compound of the formula (IV) 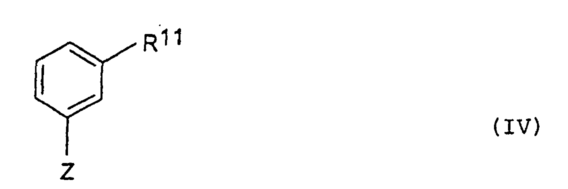 in which Z denotes MgCl, MgBr, MgI or Li and R¹¹ denotes hydrogen, O-C₁₋₆-alkyl, O-C₃₋₇-cycloalkyl or a protected hydroxy group that is present as a benzyloxy or silanyloxy group, to form a compound of the formula (IIa) 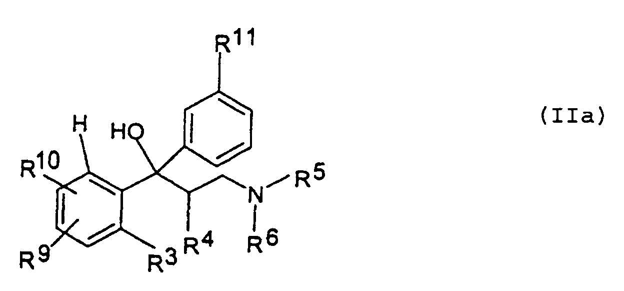 and this is then converted into the compound of the formula (II).

9. Use of the substituted amino compounds of the general formula (I) according to claim 1 as active constituent in a medicament.

10. Use according to claim 7, **characterised in that** the medicament is an analgesic.

## Revendications

1. Composés aminés substitués de formule générale (I) 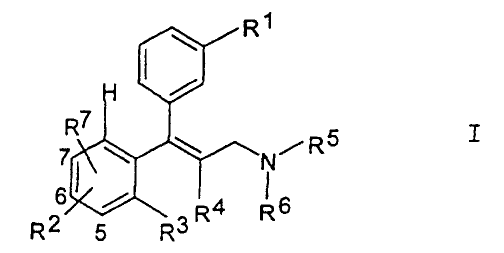 dans laquelle
R¹ représente l'hydrogène, un groupe OH, O-(alkyle en C₁ à C₆), O-(cycloalkyle en C₃ à C₇) ; (R¹ n'étant pas l'hydrogène lorsque R² et R⁷ sont tous deux de l'hydrogène, ou lorsqu'un groupe OCH₃ est substitué en position 7) ;
R² représente l'hydrogène, un groupe OH, alkyle en C₁ à C₆, aryle (aryle désignant des groupes phényle non substitués ou portant un ou plusieurs substituants OH, F, Cl, CF₃, alkyle en C₁ à C₆, alkoxy en C₁ à C₆, cycloalkoxy ayant jusqu'à 7 atomes de carbone, cycloalkyle en C₃ à C₇, alkylène en C₂ à C₆ ou phényle, les restes phényle pouvant par ailleurs être condensés, ou bien aryle désignant aussi un groupe naphtyle), un groupe Oaryle, (alcénylène en C₂ à C₆)-aryle, O-cycloalkyle en C₃ à C₇, le chlore, le fluor, un groupe alkoxy en C₁ à C₆, (alkyle en C₁ à C₆)-aryle, condensé au benzène en positions 5,6 ou 6,7, non substitué, monosubstitué ou disubstitué avec Cl, F, CF₃, alkyle en C₁ à C₆, O-(alkyle en C₁ à C₆), OH, (lorsque R¹ et R² ou R⁷ représentent l'hydrogène, R² ou R⁷ ne peut pas représenter un groupe CF₃ et R² ou R⁷ ne peut pas représenter un groupe OCH₃ en position 7), ou bien R² et R⁷ forment ensemble un groupe -O-(CH₂)₍₁₋₂₎-O- (en positions 5,6- ou 6,7) ;
R³ représente l'hydrogène ;
R⁴ est un groupe alkyle en C₁ à C₆, ou bien R³ et R⁴ forment ensemble un groupe - (CH₂)₍₁₋₄₎- ;
R⁵ est un groupe alkyle en C₁ à C₆, cycloalkyle en C₃ à C₇ ;
R⁶ est un groupe alkyle en C₁ à C₆, (alkyle en C₁ à C₆)-aryle, (alkyle en C₁ à C₆)-hétérocyclyle, le groupe hétérocyclyle désignant le furanne, le thiophène, la pyridine, la pyrimidine, le thiazole, l'oxazole, l'isoxazole, la pyridazine, la pyrazine, la quinoléine, l'isoquinoléine, la phtalazine ou la quinazoline, un groupe -CH₂-CH=C(R⁸)₂, -CH₂-(cycloalkyle en C₃ à C₇), cycloalkyle en C₃ à C₇,
R⁷ et représente l'hydrogène, un groupe OH, alkyle en C₁ à C₆, O-aryle, (alcénylène en C₂ à C₆)-aryle, O-(cycloalkyle en C₃ à C₇), alkoxy en C₁ à C₆, le chlore, le fluor, un groupe aryle, (alkyle en C₁ à C₆)-aryle, condensé au benzène en positions 5,6 ou 6,7, non substitué, monosubstitué ou disubstitué avec Cl, F, CF₃, alkyle en C₁ à C₆, O-(alkyle en C₁ à C₆), OH, (lorsque R¹ et R² ou R⁷ représentent l'hydrogène, R² ou R⁷ ne peut pas être un groupe CF₃ et R² ou R⁷ ne peut pas représenter un groupe OCH₃ en position 7),
et
R⁸ représente l'hydrogène ou un groupe CH₃,
ou leurs sels acceptables du point de vue pharmaceutique.

2. Composés aminés substitués suivant la revendication 1, **caractérisés en ce que** R⁶ est un groupe (alkyle en C₁ à C₆)-hétérocyclyle, la partie hétérocyclyle désignant le furanne, le thiophène, la pyridine, la pyrimidine, le thiazole, l'oxazole, l'isoxazole, la pyridazine, la pyrazine, la quinoléine, l'isoquinoléine, la phtalazine ou la quinazoline, ou un groupe (alkyle en C₁ à C₆)-aryle et R² et R⁷ représentent un groupe (alkyle en C₁ à C₆)-aryle, R¹, R³ à R⁵ et R⁸ ont la définition indiquée dans la revendication 1.

3. Composés aminés substitués suivant la revendication 1, **caractérisés en ce que** R² et R⁷ représentent un groupe aryle, R¹, R³ à R⁶ et R⁸ ont la définition indiquée dans la revendication 1.

4. Composés aminés substitués suivant la revendication 1, **caractérisés en ce que** R¹ représente OH ou un groupe -O-(alkyle en C₁ à C₆), R⁵ est un groupe alkyle en C₁ à C₆, R⁶ est un groupe alkyle en C₁ à C₆, R² à R⁴ et R⁷ ont la définition donnée dans la revendication 1.

5. Composés aminés substitués suivant la revendication 1, **caractérisés en ce que** R¹ représente OH, R⁵ est un groupe méthyle, R⁶ est un groupe méthyle, R² à R⁴ et R⁷ ont la définition indiquée dans la revendication 1.

6. Médicament contenant comme substance active au moins un composé suivant les revendications 1 à 5 et le cas échéant d'autres substances actives.

7. Médicament suivant la revendication 6, doué d'activité analgésique.

8. Procédé de production d'un composé de formule (I) 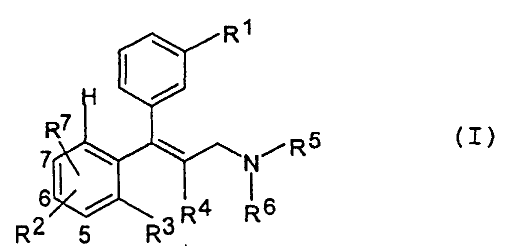 dans laquelle R¹ à R⁷ ont la définition indiquée dans la revendication 1, **caractérisé en ce qu'**on fait réagir un alcool tertiaire de formule générale (II) 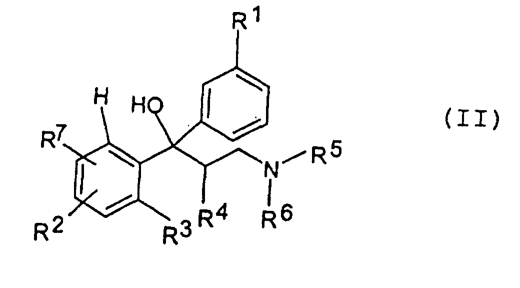 dans laquelle R¹ à R⁷ ont la même définition que dans la formule (I), dans des acides organiques ou inorganiques, dans une plage de températures comprise entre 0°C et 100°C, l'alcool tertiaire de formule générale (II) étant obtenu par le fait qu'on fait réagir tout d'abord des β-aminocétones de formule générale (III) 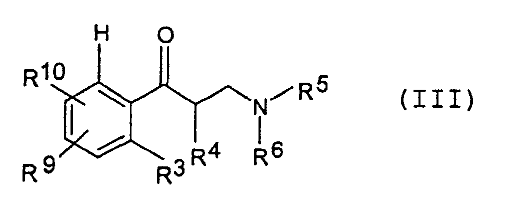 dans laquelle R³ à R⁶ ont la même définition que dans la formule (I), R⁹ est défini comme R² et R¹⁰ est défini comme R⁷, excepté que la fonction hydroxy se présente sous la forme protégée comme groupe benzyloxy ou silanyloxy, avec un composé organométallique de formule IV 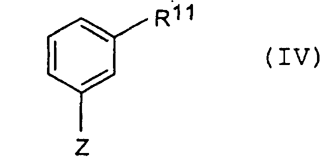 dans laquelle Z représente MgCl, MgBr, MgI ou Li et R¹¹ est l'hydrogène, un groupe O-alkyle en C₁ à C₆, O-cycloalkyle en C₃ à C₇ ou représente une fonction hydroxy protégée, qui existe comme groupe benzyloxy ou silanyloxy, pour obtenir un composé de formul 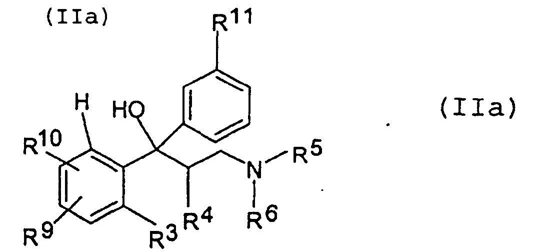 qu'on transforme ensuite en le composé de formule (II).

9. Utilisation des composés aminés substitués de formule générale (I) suivant la revendication 1 comme substance active dans un médicament.

10. Utilisation suivant la revendication 7, **caractérisée en ce que** le médicament est un analgésique.
